# EUROPEAN PATENT APPLICATION

(11) **EP 4 491 138 A1**
(43) Date of publication of application: **15.01.2025**
(21) Application number: 23815771.3
(22) Date of filing: 16.05.2023
(51) Int. Cl.: A61B 17/34, A61M 5/158

(54) **PUNCTURE NEEDLE**

(30) Priority: 31.05.2022 JP 2022089075
(71) Applicant: Terumo Kabushiki Kaisha, Tokyo 151-0072 (JP)
(72) Inventor: FUJIKI Jo, Ashigarakami-gun, Kanagawa 259-0151 (JP); KAMBARA Kayo, Ashigarakami-gun, Kanagawa 259-0151 (JP)
(74) Representative: KIPA AB
(86) International application number: PCT/JP2023/018321
(87) International publication number: WO 2023/234011

(57) **Abstract**

Provided is a puncture needle capable of, while securing echo visibility of the puncture needle, further grasping a puncture length. The puncture needle includes: a rod-shaped main body portion; a first ultrasound reflection structure formed in the main body portion; and a second ultrasound reflection structure formed in the main body portion and arranged on a proximal end side in an axial direction of the main body portion with respect to the first ultrasound reflection structure, and the second ultrasound reflection structure is arranged separated from the first ultrasound reflection structure in the axial direction.

## Description

### Technical Field

The present disclosure relates to a puncture needle.

### Background Art

JP 2010-190413 A (Patent Literature 1) discloses a puncture needle for an ultrasound endoscope in which a plurality of recesses for increasing reflection intensity of an ultrasound echo is formed on an outer peripheral surface in the vicinity of a distal end of a needle tube punctured into a biological tissue. In this puncture needle for an ultrasound endoscope, each recess includes an ultrasound reflection surface formed obliquely rearward from a surface of the needle tube toward the inside thereof, and is formed in a state where a wall of the needle tube is removed so that a space in a direction perpendicular to the ultrasound reflection surface is not blocked by the wall of the needle tube. In this puncture needle for an ultrasound endoscope, it is considered that it is possible to obtain a clear ultrasound echo image of the needle tube by effectively increasing the ultrasound echo of the distal end portion of the needle tube punctured into the biological tissue.

JP 2011-125632 A (Patent Literature 2) discloses an ultrasound-guided puncture needle for performing puncture while detecting a position using reflection of ultrasound and an indwelling needle having the same. This puncture needle has groove portions reflecting ultrasound on an outer peripheral surface. The groove portions include a first groove portion provided in a portion of the outer peripheral surface on a back side of a blade surface, and a second groove portion provided in a portion of the outer peripheral surface in the vicinity of a distal end portion where the blade surface is formed. A plurality of the first groove portions, each of which extends in a circumferential direction and has both ends facing the blade surface, is provided in an axial direction of the puncture needle. With this puncture needle, it is considered to be possible to more effectively reflect ultrasound, so that a position in a body can be reliably confirmed with high accuracy.

### Citation List

### Patent Literature

Patent Literature 1: JP 2010-190413 A
Patent Literature 2: JP 2011-125632 A

### Summary of Invention

### Technical Problem

When a procedure such as puncture with a puncture needle is performed under an echo, it is important to accurately grasp a position of a needle tip in order to improve accuracy of the puncture. Moreover, when a puncture length (a depth of puncture of the puncture needle) can be grasped, it is possible to prevent a surrounding tissue from being unexpectedly damaged.

However, in the conventional technology, although the position of the puncture needle can be confirmed by the echo, it is difficult to grasp the puncture length by the echo.

The present disclosure has been made in view of such circumstances, and an object thereof is to provide a puncture needle capable of, while securing echo visibility of the puncture needle, further grasping a puncture length.

### Solution to Problem

A puncture needle according to the present disclosure for achieving the above-described object includes:
a rod-shaped main body portion;
a first ultrasound reflection structure formed in the main body portion; and
a second ultrasound reflection structure formed in the main body portion and arranged on a proximal end side in an axial direction of the main body portion with respect to the first ultrasound reflection structure, and
the second ultrasound reflection structure is arranged separated from the first ultrasound reflection structure in the axial direction.

Moreover, in the puncture needle according to the present disclosure,
the main body portion may include a rod-shaped body portion and a needle tip portion arranged at a distal end of the body portion,
the first ultrasound reflection structure may be arranged at the needle tip portion, and
the second ultrasound reflection structure may be arranged at the body portion.

Moreover, in the puncture needle according to the present disclosure,
the first ultrasound reflection structure may be arranged at a distal end portion of the needle tip portion.

Moreover, in the puncture needle according to the present disclosure,
the second ultrasound reflection structure may be a spiral groove formed on an outer peripheral surface of the body portion.

Moreover, in the puncture needle according to the present disclosure,
the needle tip portion may have a pyramid shape.

Moreover, in the puncture needle according to the present disclosure,
the body portion may be formed in a cylindrical shape, and
the needle tip portion may have an opening that communicates an internal space and an external space of the body portion.

### Advantageous Effects of Invention

It is possible to provide a puncture needle capable of, while securing echo visibility of the puncture needle, further grasping a puncture length.

### Brief Description of Drawings

Fig. 1 is a perspective view of a puncture needle according to a first embodiment.
Fig. 2 is a side view of a needle tip portion of the puncture needle according to the first embodiment.
Fig. 3 is a view for describing an aspect of reflection of ultrasound in a first ultrasound reflection structure (first structure).
Fig. 4 is an explanatory view of the first structure configured by transverse grooves.
Fig. 5 is an explanatory view of the first structure configured by intersection grooves.
Fig. 6 is an explanatory view of the first structure configured by circular recesses.
Fig. 7 is an explanatory view of the first structure configured by triangular recesses.
Fig. 8 is a view for describing an aspect of reflection of ultrasound in a second ultrasound reflection structure (reflection structure portion).
Fig. 9 is an explanatory view of the reflection structure portion configured by a spiral groove.
Fig. 10 is an explanatory view of the reflection structure portion configured by an annular groove.
Fig. 11 is an explanatory view of the reflection structure portion configured by a plurality of the annular grooves.
Fig. 12 is an explanatory view of the reflection structure portion configured by a dotted-line circular groove.
Fig. 13 is an explanatory view of the reflection structure portion configured by an annular oblique line groove.
Fig. 14 is an explanatory view of the reflection structure portion configured by an arc array groove.
Fig. 15 is a side view of a needle tip portion of a puncture needle according to a second embodiment as viewed from a side of a blade surface.
Fig. 16 is a side view of the needle tip portion of the puncture needle according to the second embodiment as viewed from a side opposite to the blade surface.
Fig. 17 is an explanatory view in a case where a first structure configured by V-shaped grooves is formed on an exposed inner surface.
Fig. 18 is an explanatory view in a case where the first structure configured by circular recesses is formed on the exposed inner surface.
Fig. 19 is an explanatory view in a case where the first structure configured by grooves formed from a first blade surface portion to a second blade surface portion through the exposed inner surface is formed on the exposed inner surface.
Fig. 20 is an explanatory view in a case where the first structure configured by a triangular recess is formed on the exposed inner surface.
Fig. 21 is an explanatory view in a case where the first structure configured by a groove along a spiral shape is formed on an outer surface of the needle tip portion.
Fig. 22 is an explanatory view in another case where the first structure configured by a groove along a spiral shape is formed on the outer surface of the needle tip portion.
Fig. 23 is an explanatory view in a case where a plurality of grooves along a circumferential direction of a body portion is arranged at equal intervals along an axial direction to configure the first structure 4.
Fig. 24 is a side view of a puncture needle according to a third embodiment.

### Description of Embodiments

A puncture needle according to an embodiment of the present disclosure will be described with reference to the drawings.

### (First Embodiment)

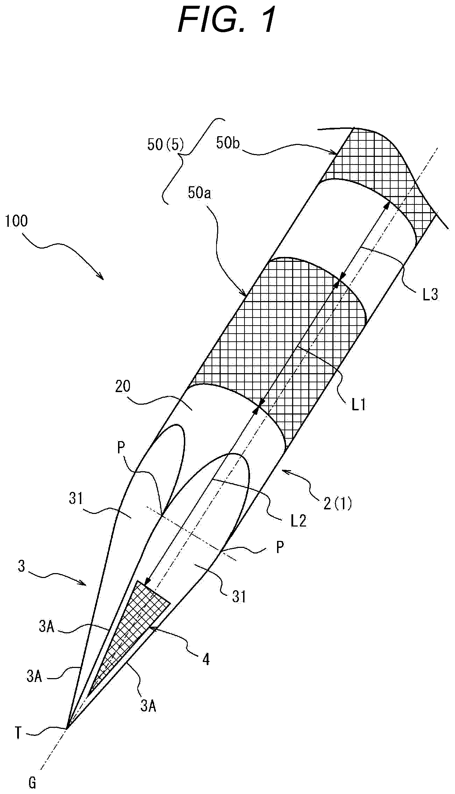

Fig. 1 illustrates a puncture needle 100 according to the present embodiment. Fig. 1 is a perspective view of side surfaces of a main body portion 1 as viewed from a distal end side of the puncture needle 100 in an oblique direction relative to an axial center G of the main body portion 1.

The puncture needle 100 includes the rod-shaped main body portion 1, a first ultrasound reflection structure 4 formed in the main body portion 1, and a second ultrasound reflection structure 5 formed in the main body portion 1 and arranged on a proximal end side in an axial direction of the main body portion 1 (direction along the axial center G of the main body portion 1) with respect to the first ultrasound reflection structure 4.

The second ultrasound reflection structure 5 is arranged separated from the first ultrasound reflection structure 4 in the axial direction. The first ultrasound reflection structure 4 and the second ultrasound reflection structure 5 will be described later.

The main body portion 1 includes a rod-shaped body portion 2 and a needle tip portion 3 arranged at a distal end of the body portion 2. In the present embodiment, an axial center of the body portion 2 overlaps with the axial center G of the main body portion 1, and an axial direction of the body portion 2 is the same as the axial direction of the main body portion 1. Similarly, a circumferential direction (circumferential direction centered on the axial direction) of the main body portion 1 is the same as a circumferential direction of the body portion 2. In the present embodiment, the needle tip portion 3 is the distal end portion of the body portion 2. Hereinafter, the axial direction of the main body portion 1 and the same direction as the axial direction of the main body portion 1 may be simply referred to as the axial direction. Furthermore, the circumferential direction of the main body portion 1 and the same direction as the circumferential direction of the main body portion 1 may be simply referred to as the circumferential direction.

The main body portion 1 can be formed of, for example, a metal alloy such as stainless steel, a titanium alloy, or a cobalt-chromium alloy, or a fluororesin such as polytetrafluoroethylene, or a resin such as nylon. The main body portion 1 may be solid, or may have a cylindrical shape in which a space continuously formed along the axial center G is formed inside the main body portion 1.

The body portion 2 has, for example, a rod shape and a columnar shape. The body portion 2 may be solid or hollow (for example, a cylindrical shape). In the present embodiment, a case where the body portion 2 is hollow will be described below as an example.

As described above, the needle tip portion 3 is arranged at the distal end of the body portion 2. The needle tip portion 3 has, for example, a pyramid shape. Examples of the pyramid shape include a conical shape or a polygonal pyramid shape including a plurality of planar portions on side surfaces. Examples of the polygonal pyramid shape include a triangular pyramid shape, a quadrangular pyramid shape, and a pentagonal or more pyramid shape. Fig. 1 illustrates a case where the needle tip portion 3 has a triangular pyramid shape as an example. That is, the needle tip portion 3 in the present embodiment has three planar portions 31 on side surfaces thereof. In the axial direction of the main body portion 1, the body portion 2 on a distal end side with respect to a proximal end portion on the most proximal end side among proximal end portions of the respective planar portions 31 is the needle tip portion 3.

The needle tip portion 3 having a polygonal pyramid shape such as a triangular pyramid shape can be formed by, for example, cutting or polishing a distal end of a rod-shaped material, which is to serve as the main body portion 1, to form planar portions which are to serve as the planar portions 31 of the polygonal pyramid. Corners of the side surfaces of the needle tip portion 3, that are, boundary portions between the adjacent planar portions 31 and 31 of the side surfaces in the polygonal pyramid (the triangular pyramid in the present embodiment) shape form blades 3A extending from a vertex T (a distal end of the puncture needle 100) toward the side surface portion of the main body portion 1.

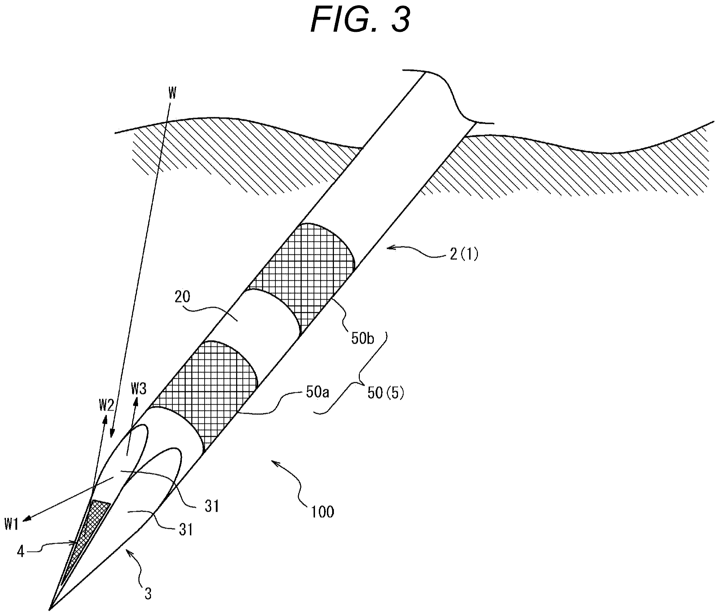

The first ultrasound reflection structure 4 (hereinafter referred to as the first structure 4) is a stereoscopic ultrasound reflection structure that changes a reflection direction and intensity of ultrasound W (see Fig. 3) emitted by an ultrasound image diagnosis device (hereinafter may be referred to as an echo device). The reflection of the ultrasound W in the first structure 4 will be described later.

The first structure 4 is arranged at the needle tip portion 3. The first structure 4 is preferably arranged at a distal end portion of the needle tip portion 3 as described later.

The first structure 4 is preferably arranged in a predetermined region of a surface of at least one of the planar portions 31 in the needle tip portion 3. As a result, echo visibility of the needle tip portion 3 is improved. Since the echo visibility of the needle tip portion 3 is improved, accuracy of puncture in a procedure is improved.

The first structure 4 may be arranged on all the planar portions 31, or may be arranged on a part of all the planar portions 31. Since the needle tip portion 3 includes the planar portion 31 in which the first structure 4 is arranged and the planar portion 31 in which the first structure 4 is not arranged, it may be possible to grasp a direction in the circumferential direction (direction relative to a body surface, that is, a probe of the echo device) of the main body portion 1 (puncture needle 100) under an echo (image diagnosis by the echo device).

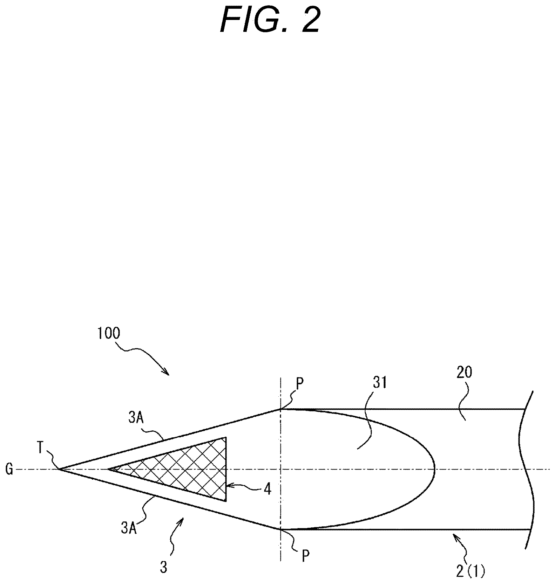

As illustrated in Fig. 2, in a case where the needle tip portion 3 is viewed in a side view of the main body portion 1 (top view along a radial direction of the main body portion 1), the planar portion 31 includes a triangular region positioned on a distal end side and a region that has a partial elliptical or parabolic shape and is positioned on a proximal end side with respect to the triangular region. In Figs. 1 and 2, points, positioned at a boundary between the triangular region and the region having the partial elliptical or parabolic shape in an outer peripheral shape of the planar portion 31, are illustrated as boundary points P and P.

In particular, the first structure 4 is preferably arranged at a distal end portion of the planar portion 31 (that is, the distal end portion of the needle tip portion 3). As a result, echo visibility of the distal end portion of the needle tip portion 3 is improved. Since the echo visibility of the distal end portion of the needle tip portion 3 is improved, the accuracy of the puncture in the procedure is further improved. Specifically, the first structure 4 is preferably arranged on the distal end side with respect to a line connecting the boundary points P and P. Note that the first structure 4 may extend from the distal end side with respect to the line connecting the boundary points P and P to the proximal end side with respect to the line connecting the boundary points P and P.

Note that the first structure 4 is preferably formed so as to avoid the vicinity of the blades 3A in the planar portions 31. As a result, it is possible to maintain sharpness of the blades 3A at the time of puncture with the puncture needle 100 and ensure favorable puncture performance.

Due to the stereoscopic shape thereof, the first structure 4 can reflect the ultrasound W incident from the probe (not illustrated) or the like of the echo device as a reflected wave W2 having high intensity to some extent in a direction different from a direction of a reflected wave W1 reflected at the highest intensity by a region of the planar portion 31 where the first structure 4 is not formed, as illustrated in Fig. 3.

As an example, the first structure 4 can reflect the reflected wave W2 in a direction along an incident direction of the ultrasound W at the intensity stronger than that of a reflected wave W3 which is a reflected wave of the incident ultrasound W, reflected in a direction along the incident direction of the ultrasound W from the region of the planar portion 31 where the first structure 4 is not formed.

That is, since the first structure 4 is arranged in the planar portion 31, as illustrated in Fig. 3, when a procedure such as puncture is performed through echo observation (that is, under an echo) from a proximal end side (a side of a body surface of a living body punctured with the puncture needle 100) of the puncture needle 100 (main body portion 1), the echo visibility of the needle tip portion 3 is improved, whereby a position of a needle tip can be accurately grasped by the echo, and the accuracy of the puncture in the procedure is improved.

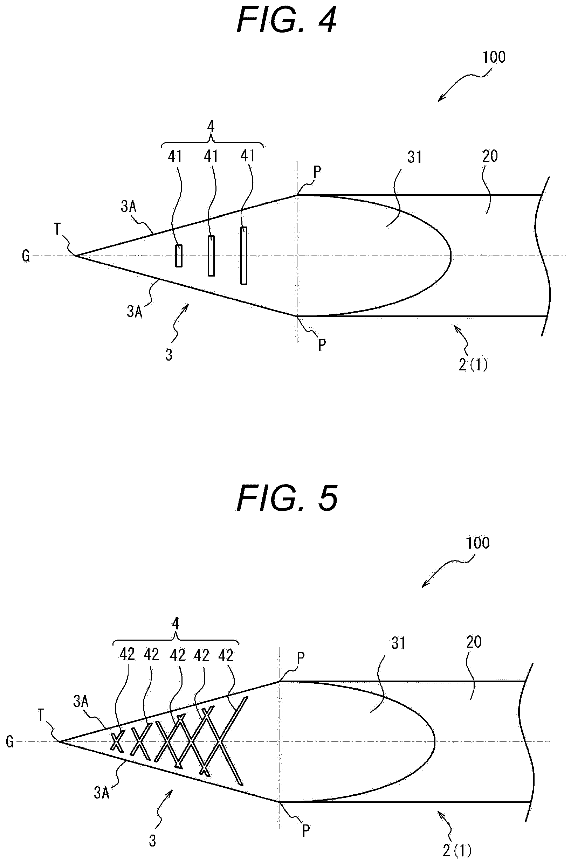

The first structure 4 may have a stereoscopic shape such as an uneven shape as a structure that changes a direction and intensity in which the ultrasound W (see Fig. 3) is reflected. The uneven shape is, for example, a shape obtained by combining a dent, a recess, a groove, and the like. A specific example of the first structure 4 is a groove shape (see, for example, Fig. 4). A formation method or a processing method of the first structure 4 is not limited, and laser processing, cutting, rolling, pressing, or the like can be employed. When the first structure 4 has the groove shape, for example, a groove width can be 30 µm or more and 100 µm or less (65 µm as an example).

Fig. 4 illustrates a case where, as the first structure 4, a plurality of (three in the present embodiment) transverse grooves 41 formed in a direction orthogonal to the axial center G is formed on the planar portion 31. The transverse grooves 41 are arranged at equal intervals, for example, along the axial direction. In the respective transverse grooves 41, a length of the transverse groove 41 positioned on the proximal end side is longer than a length of the transverse groove 41 positioned on the distal end side in the direction intersecting the axial center G. Since the transverse grooves 41 are formed in the direction orthogonal to the axial center G, ultrasound, emitted as an echo from the side of the body surface of the living body punctured with the puncture needle 100, is reflected to the side of the body surface at strong intensity when the procedure such as puncture is performed through the echo observation from the proximal end side of the puncture needle 100, so that the echo visibility is improved, whereby the position of the needle tip can be accurately grasped by the echo.

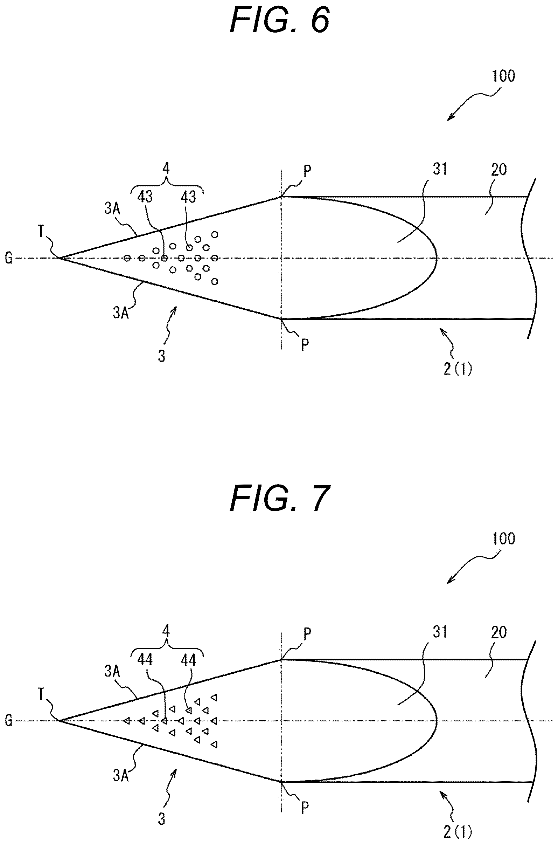

Other modifications of the first structure 4 are intersecting groove shapes (see Fig. 5), a large number of small recess shapes (for example, dimpled or engraved shapes, see Figs. 6 and 7), a rough surface due to blasting, and the like.

As illustrated in Fig. 5, the first structure 4 may be formed by a plurality of intersection grooves 42 intersecting each other. In the example illustrated in Fig. 5, the respective intersection grooves 42 intersect relative to the axial direction. The respective intersection grooves 42 may intersect one or more other intersection grooves 42 to form a lattice shape.

As illustrated in Fig. 6, the first structure 4 may be formed by arranging a plurality of circular recesses 43 as dimpled recesses. In the example illustrated in Fig. 6, a case where the first structure 4 is formed by arranging the circular recesses 43 in a triangular zigzag lattice shape is illustrated.

As illustrated in Fig. 7, the first structure 4 may be formed by arranging a plurality of triangular recesses 44 as examples of engrave-shaped recesses. In the example illustrated in Fig. 7, a case where the first structure 4 is formed by arranging the triangular recesses 44 in a triangular zigzag lattice shape is illustrated.

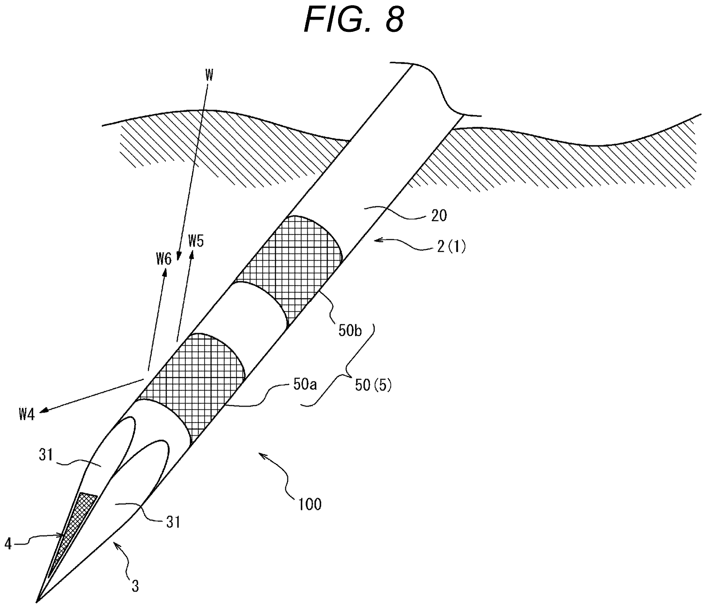

As illustrated in Fig. 1, the second ultrasound reflection structure 5 (hereinafter referred to as the second structure 5) is a stereoscopic ultrasound reflection structure that changes the reflection direction and intensity of the ultrasound W (see Fig. 8) emitted by the echo device. The reflection of the ultrasound W in the second structure 5 will be described later.

The second structure 5 may be arranged at the body portion 2. The second structure 5 is formed on an outer surface 20 which is a surface on a radially outer side (outer peripheral surface) of the body portion 2. The second structure 5 is preferably arranged at a predetermined distance separated from the first structure 4 in the axial direction. As a result, a positional relationship between the first structure 4 and the second structure 5 can be easily grasped under an echo, and while securing echo visibility of the puncture needle 100, a puncture length can be further grasped. The distance by which the first structure 4 and the second structure 5 are separated from each other is preferably a distance by which the first structure 4 and the second structure 5 can be separated and observed on an echo or more (for example, 0.6 mm or more).

An extension length of the second structure 5 in the axial direction (a distance from a distal end to a proximal end of the second structure 5 in the axial direction) may be longer than a separation distance L2 between the first structure 4 and the second structure 5. As a result, echo visibility of the second structure 5 is improved. Since the echo visibility of the second structure 5 is improved, a position of the body portion 2 can be accurately grasped, and the accuracy of the puncture in the procedure is further improved. Furthermore, under an echo, discriminability between the first structure 4 and the second structure 5 may be improved.

The second structure 5 may include a plurality of ultrasound reflection structures separated in the axial direction (hereinafter referred to as reflection structure portions 50). Fig. 1 illustrates a case where the second structure 5 includes a first reflection structure portion 50a (an example of the reflection structure portions) which is the reflection structure portion 50 arranged on a distal end side and a second reflection structure portion 50b which is the reflection structure portion 50 arranged on a proximal end side with respect to the first reflection structure portion 50a and adjacent to the first reflection structure portion 50a. Although not illustrated, the second structure 5 may further include another reflection structure portion 50 in addition to the first reflection structure portion 50a and the second reflection structure portion 50b. That is, the second structure 5 may include three or more reflection structure portions 50.

The reflection structure portion 50 adjacent to the first structure 4 in the axial direction is preferably arranged at a predetermined distance separated from the first structure 4 in the axial direction. As a result, a positional relationship between the first structure 4 and the reflection structure portion 50 can be easily grasped under an echo, and while securing the echo visibility of the puncture needle 100, the puncture length can be further appropriately grasped. As a result, the accuracy of the puncture in the procedure is improved.

An extension length L1 of the reflection structure portion 50 in the axial direction may be longer than the separation distance L2 between the first structure 4 and the reflection structure portion 50. As a result, echo visibility of the reflection structure portion 50 is improved. Furthermore, under an echo, discriminability between the first structure 4 and the reflection structure portion 50 may be improved. As a result, the accuracy of the puncture in the procedure is improved. The extension length L1 may preferably be, for example, 5 mm or more and 10 mm or less, but is not limited thereto.

Each of the reflection structure portions 50 adjacent in the axial direction is preferably arranged at a predetermined distance separated from the adjacent reflection structure portion 50 in the axial direction. As a result, a positional relationship between the adjacent reflection structure portions 50 can be easily grasped under an echo, and while securing the echo visibility of the puncture needle 100, the puncture length can be further appropriately grasped. As a result, the accuracy of the puncture in the procedure is improved.

The extension length L1 of the reflection structure portion 50 in the axial direction may be longer than a separation distance L3 between the adjacent reflection structure portions 50. As a result, the echo visibility of each reflection structure portion 50 may be improved, and discriminability from the adjacent reflection structure portion 50 may be improved. As a result, the accuracy of the puncture in the procedure is improved.

The reflection structure portion 50 may be formed in an annular shape along the circumferential direction on the outer surface 20 of the body portion 2, or may be formed in a part along the circumferential direction. In a case where the reflection structure portion 50 is formed in an annular shape along the circumferential direction on the outer surface 20 of the body portion 2, it may be preferable since it can be visually recognized by the echo device regardless of a direction of the main body portion 1 (puncture needle 100) in the circumferential direction. In a case where the reflection structure portion 50 is formed in a part along the circumferential direction of the outer surface 20 of the body portion 2, the direction of the main body portion 1 (puncture needle 100) in the circumferential direction may be grasped. As a result, the accuracy of the puncture in the procedure is improved.

Due to the stereoscopic shape thereof, the reflection structure portion 50 can reflect the ultrasound W incident from the probe (not illustrated) or the like of the echo device as a reflected wave W6 having high intensity to some extent in a direction different from a direction of a reflected wave W4 reflected at the highest intensity by the outer surface 20 of the body portion 2 where the second structure 5 is not formed, as illustrated in Fig. 8.

As an example, the reflection structure portion 50 can reflect the reflected wave W6 in a direction along an incident direction of the ultrasound W at the intensity stronger than that of a reflected wave W5 which is a reflected wave of the incident ultrasound W, reflected in a direction along the incident direction of the ultrasound W from the outer surface 20 of the body portion 2 where the first structure 4 is not formed.

That is, since the second structure 5 (reflection structure portion 50) is arranged on the outer surface 20 of the body portion 2, as illustrated in Fig. 8, when a procedure such as puncture is performed through echo observation (that is, under an echo) from the proximal end side (the side of the body surface of the living body punctured with the puncture needle 100) of the puncture needle 100 (main body portion 1), the echo visibility is improved, whereby the position of the body portion 2 can be accurately grasped by the echo, and the accuracy of the puncture in the procedure is improved.

Note that, in Fig. 8, the first reflection structure portion 50a is illustrated as an example of the reflection structure portion 50, but the same applies to the second reflection structure portion 50b. That is, the puncture length can be grasped by grasping the positional relationship between each reflection structure portion 50 (second structure 5) and the first structure 4.

The reflection structure portion 50 may have a stereoscopic shape such as an uneven shape as a structure that changes a direction and intensity in which the ultrasound W (see Fig. 8) is reflected. The uneven shape is, for example, a shape obtained by combining a dent, a recess, a groove, and the like. A formation method or a processing method of the reflection structure portion 50 is not limited, and laser processing, cutting, rolling, pressing, or the like can be employed. From a viewpoint of processability, the second structure 5 preferably has a groove shape. Various shapes can be employed as a pattern of a groove shape of the reflection structure portion 50. When the reflection structure portion 50 has the groove shape, for example, a groove width can be 20 µm or more and 100 um or less (40 um as an example).

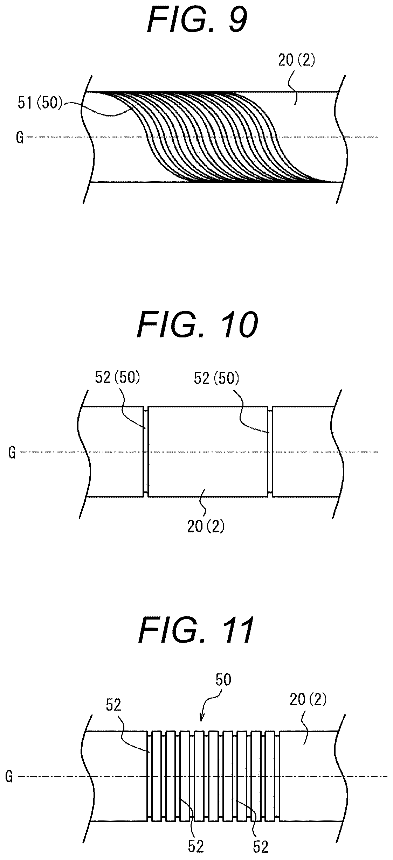

Fig. 9 illustrates a case where a spiral groove 51 extending in the axial direction along the circumferential direction of the body portion 2 is formed as the reflection structure portion 50. The spiral groove 51 is formed so as to make two or more turns around the outer surface 20 of the body portion 2 along the circumferential direction. In the case where the spiral groove 51 is formed in the body portion 2 to form the reflection structure portion 50, since a surface portion where no groove is formed along the spiral groove 51 is formed on the outer surface 20 of the body portion 2, the body portion 2 may be hardly broken even when an external force intersecting the axial direction is applied to the body portion 2. When the reflection structure portion 50 is the spiral groove 51, for example, a pitch can be 20 µm or more and 1,000 µm or less (100 µm as an example).

As other modifications in a case where the reflection structure portion 50 has a groove shape, the following can be exemplified.

For example, there is a case where an annular groove 52 which makes one turn in the circumferential direction of the body portion 2 (one turn circle, see Fig. 10) is provided and a case where a plurality of the annular grooves 52 is arranged adjacent to each other in the axial direction (see Fig. 11). In the case of Fig. 10, each annular groove 52 is another reflection structure portion 50. In the case of Fig. 11, the plurality of annular grooves 52 configures one reflection structure portion 50.

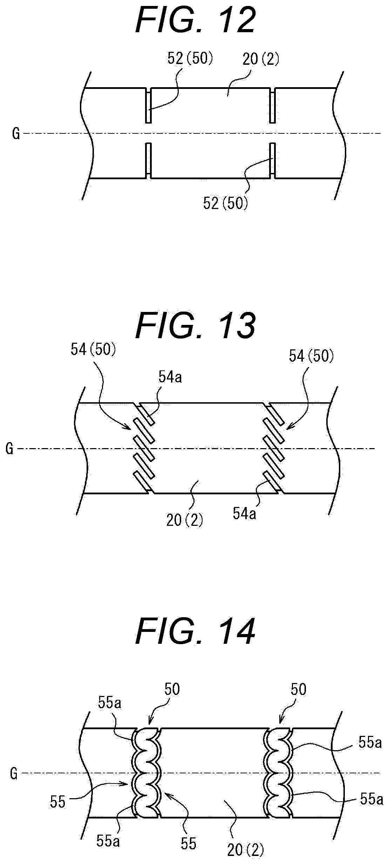

Furthermore, there is a case where a dotted-line circular groove 53 which is formed along the circumferential direction and in which at least one part of an annular shape is interrupted (see Fig. 12) is provided and a case where a plurality of the dotted-line circular grooves 53 is arranged adjacent to each other in the axial direction (not illustrated). In the case of Fig. 12, each dotted-line circular groove 53 is another reflection structure portion 50.

Furthermore, there is a case where an annular oblique line groove 54 in which short oblique line grooves 54a intersecting the circumferential direction and the axial direction are arrayed in an annular shape along the circumferential direction (see Fig. 13) is provided and a case where a plurality of the annular oblique line grooves 54 is arranged adjacent to each other in the axial direction (not illustrated). In the case of Fig. 13, each annular oblique line groove 54 is another reflection structure portion 50.

Furthermore, for example, there is a case where two arc array grooves 55 formed in an annular shape by arranging a plurality of arc-shaped small grooves 55a in the circumferential direction are arranged adjacent to each other in the axial direction (see Fig. 14). In the case of Fig. 14, the two arc array grooves 55 configure one reflection structure portion 50.

In addition to the groove shape, the reflection structure portion 50 may be formed in a large number of small recess shapes, a rough surface by blasting, or the like as in the first structure 4.

### (Second Embodiment)

In the first embodiment, the case where the needle tip portion 3 has the pyramid shape has been described. A second embodiment is different in that a needle tip portion 3 has a blade surface portion 6 in which a blade surface 60 intersecting an axial center G is formed, and other points are similar. Hereinafter, differences from the first embodiment will be mainly described, and description of common portions with the first embodiment will be appropriately omitted.

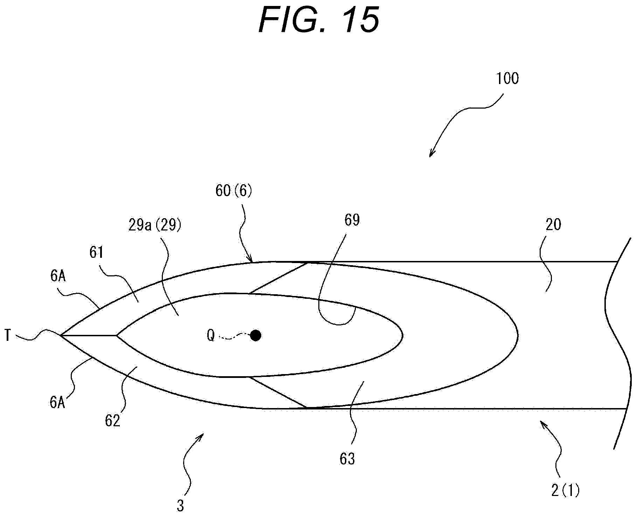

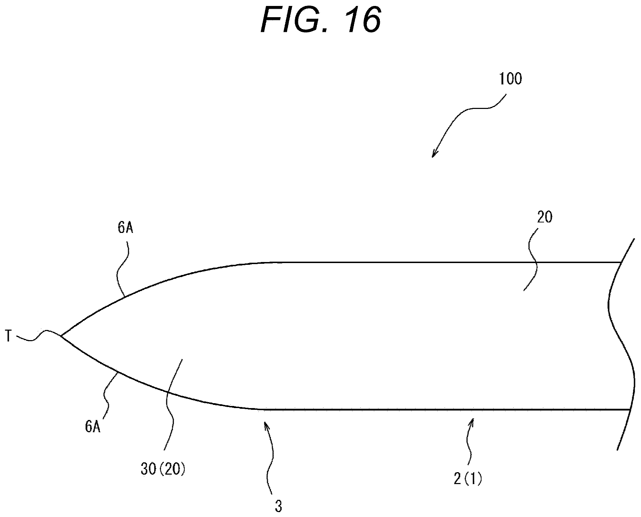

Figs. 15 and 16 illustrate the needle tip portion 3 of a puncture needle 100 according to the present embodiment in a side view (radial direction view). Fig. 15 is a side view of the needle tip portion 3 as viewed from a side of the blade surface 60 of the blade surface portion 6. Fig. 16 is a side view of the needle tip portion 3 as viewed from a side opposite to the side of the blade surface 60 of the blade surface portion 6. In the present embodiment, a body portion 2 on a distal end side with respect to a proximal end portion of the blade surface 60 in the axial direction of a main body portion 1 is the needle tip portion 3.

As illustrated in Fig. 15, the blade surface portion 6 includes a first blade surface portion 61, a second blade surface portion 62, and a third blade surface portion 63 as the blade surface 60. The third blade surface portion 63 is a surface portion arranged on a proximal end side of the first blade surface portion 61 or the second blade surface portion 62, and is arranged, in the present embodiment, on the proximal end sides of the first blade surface portion 61 and the second blade surface portion 62. In Fig. 15, a case where the puncture needle 100 is a so-called lancet type is illustrated as an example. The blade surface 60 is formed with an opening 69 that allows an internal space and an external space of the body portion 2 to communicate with each other. In the puncture needle 100 of the present embodiment, a part of an inner surface 29, which is a surface of the inside of a cylinder of the body portion 2, is exposed to the outside through the opening 69. Hereinafter, in the inner surface 29, a surface on a distal end side with respect to a proximal end portion of the opening 69 in the axial direction of the main body portion 1 is referred to as an exposed inner surface 29a.

The first blade surface portion 61 and the second blade surface portion 62 are surface portions arranged on a distal end side with respect to the third blade surface portion 63. The first blade surface portion 61 is arranged on a right side when the distal end side is viewed from the third blade surface portion 63, and the second blade surface portion 62 is arranged on a left side when the distal end side is viewed from the third blade surface portion 63. The first blade surface portion 61 and the second blade surface portion 62 are at the same position in a direction along the axial center G. The first blade surface portion 61 and the second blade surface portion 62 are inclined toward an outer side of the cylinder relative to the third blade surface portion 63. That is, the first blade surface portion 61 is inclined to the right side. Furthermore, the second blade surface portion 62 is inclined to the left side. End portions of the first blade surface portion 61 and the second blade surface portion 62 on the radially outer side in the main body portion 1 are blades 6A extending from a vertex T toward a side surface portion of the main body portion 1.

For example, a first structure 4 may be arranged on the blade surface 60, the exposed inner surface 29a, or an outer surface 30 (a portion of the outer surface 20, positioned at the needle tip portion 3) of the needle tip portion 3 of the body portion 2. More specifically, the first structure 4 may be arranged at only one part, only two parts, or all of the blade surface 60, the exposed inner surface 29a, or the outer surface 30. The first structure 4 does not need to be formed on an entire surface of each surface of the blade surface 60, the exposed inner surface 29a, or the outer surface 30, and it is sufficient that the first structure 4 is formed only on a part of each surface. The first structure 4 is preferably arranged at a distal end portion of the needle tip portion 3 as in the first embodiment. As a result, echo visibility of the distal end portion of the needle tip portion 3 is improved. More preferably, as illustrated in Fig. 15, the first structure 4 is preferably arranged on the distal end side with respect to a center Q of the opening 69 in the axial direction in the side view in which the needle tip portion 3 is viewed from the side of the blade surface 60 of the blade surface portion 6. As a result, the echo visibility of the distal end portion of the needle tip portion 3 is improved. Since the echo visibility of the distal end portion of the needle tip portion 3 is improved, accuracy of a puncture in a procedure is further improved. Note that the first structure 4 may extend from the distal end side with respect to the center Q to the proximal end side with respect to the center Q.

As in the case of the first embodiment, the first structure 4 can be formed as a groove shape, a dimpled shape, or a rough surface by blasting.

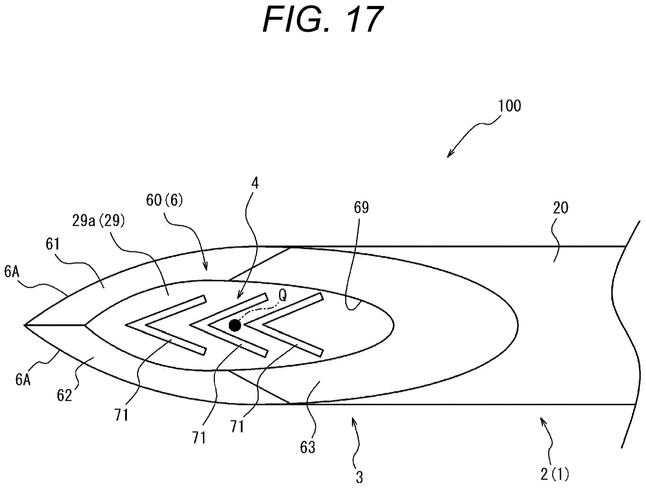

Fig. 17 illustrates a case where a plurality of (three in Fig. 17) V-shaped grooves 71 having V-shapes in which vertexes of valleys are positioned on the distal end side is formed on the exposed inner surface 29a to configure the first structure 4. The plurality of V-shaped grooves 71 is arranged at equal intervals, for example, along the axial direction.

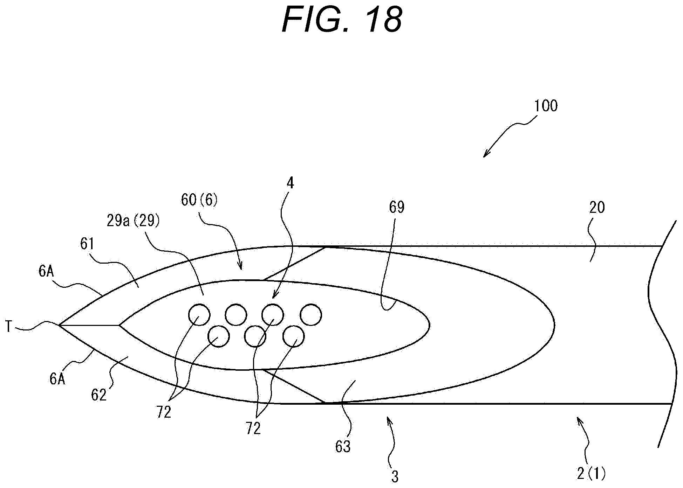

Fig. 18 illustrates a case where a plurality of circular recesses 72 as dimpled recesses is formed on the exposed inner surface 29a to configure the first structure 4. The circular recesses 72 may be arranged in, for example, a triangular zigzag lattice shape.

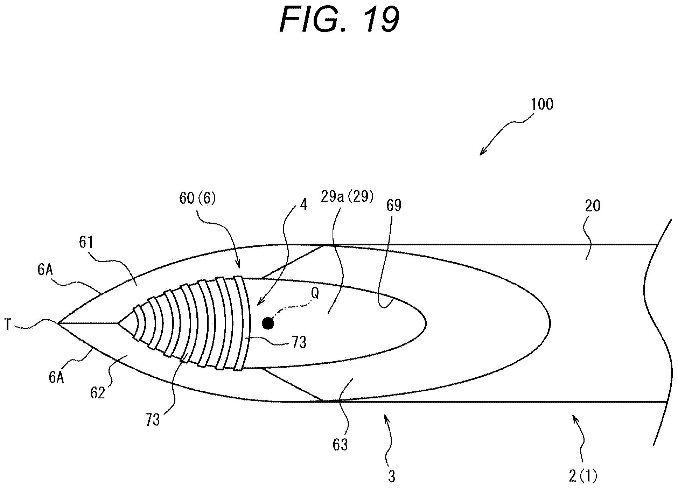

Fig. 19 illustrates a case where a plurality of grooves 73 along the circumferential direction of the body portion 2 is formed from an inner end portion of the first blade surface portion 61 (a radially inner end portion of the body portion 2) to an inner end portion of the second blade surface portion 62 through the exposed inner surface 29a to configure the first structure 4. The plurality of grooves 73 is arranged at equal intervals, for example, along the axial direction. In this example, the first structure 4 is arranged on the distal end side with respect to the center Q. Furthermore, the grooves 73 as the first structure 4 are formed so as to avoid the vicinity of the blades 6A. As a result, it is possible to maintain sharpness of the blades 6A at the time of puncture with the puncture needle 100 and ensure favorable puncture performance.

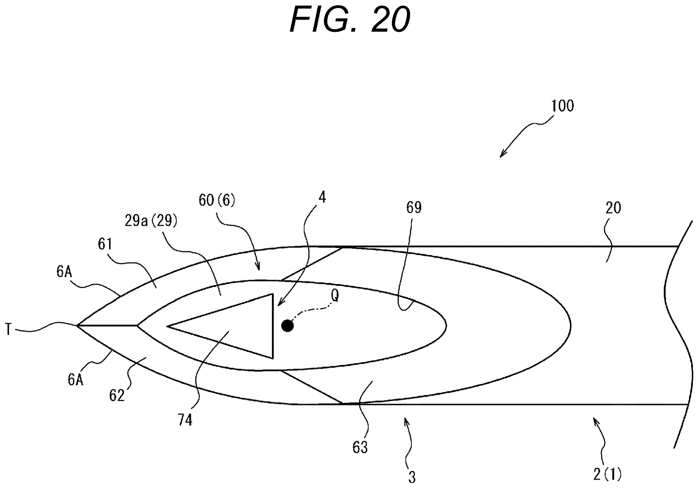

Fig. 20 illustrates a case where, as the first structure 4, a triangular recess 74 having an isosceles triangular shape with one of vertexes positioned on the distal end side and a base along the circumferential direction is formed in the exposed inner surface 29a. In this example, the first structure 4 is arranged on the distal end side with respect to the center Q.

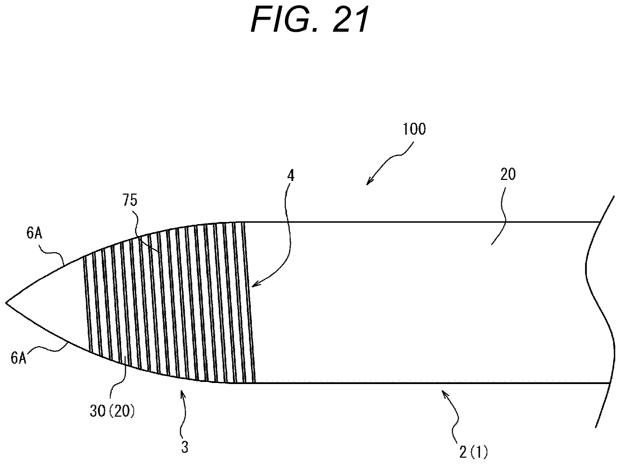

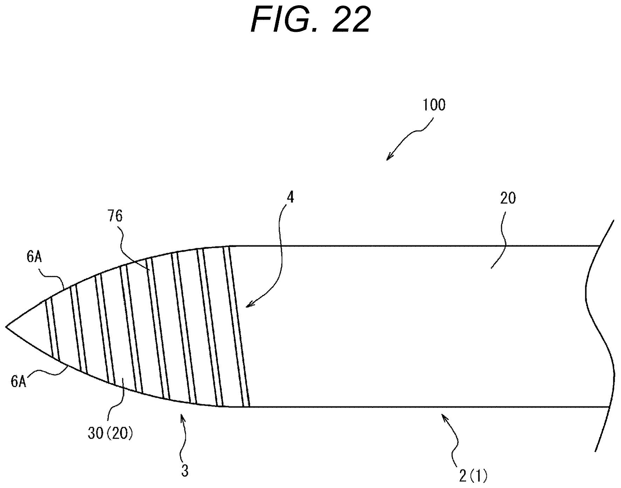

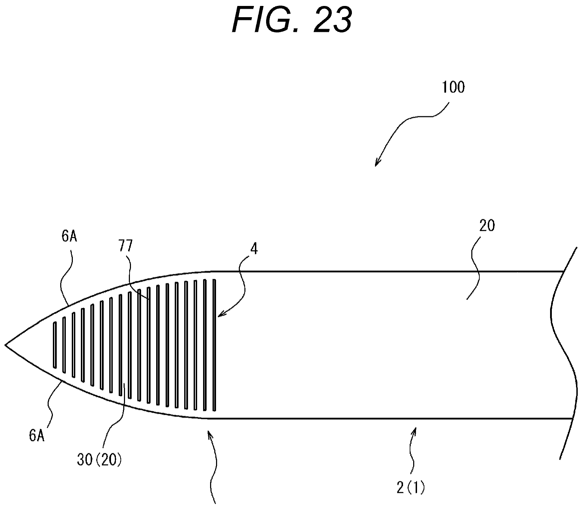

Figs. 21 to 23 illustrate a case where the first structure 4 is formed on the outer surface 30. Figs. 21 and 22 illustrate a case where grooves 75 and 76 are formed on the outer surface 30 along spiral shapes extending in the axial direction along the circumferential direction of the body portion 2 to configure the first structure 4. The groove 76 illustrated in Fig. 22 has a wider pitch (a wider interval along the axial direction) than the groove 75 illustrated in Fig. 21. Fig. 23 illustrates a case where a plurality of grooves 77 along the circumferential direction of the body portion 2 is arranged at equal intervals, for example, along the axial direction to configure the first structure 4. In the example illustrated in Fig. 23, the grooves 77 are formed so as to avoid the vicinity of the blades 6A. The grooves 75, 76, and 77 can have a groove width of, for example, 20 µm or more and 100 µm or less (40 µm as an example). Furthermore, when a plurality of the grooves 75 and 76 and the plurality of the grooves 77 are formed, a pitch can be 50 µm or more and 300 µm or less (100 µm as an example).

### (Third Embodiment)

In the first embodiment, the case where the needle tip portion 3 has the pyramid shape, particularly the triangular pyramid shape has been described. A third embodiment is different in that a needle tip portion 3 has a conical shape, and other points are similar. Hereinafter, differences from the first embodiment will be mainly described, and description of common portions with the first embodiment will be appropriately omitted.

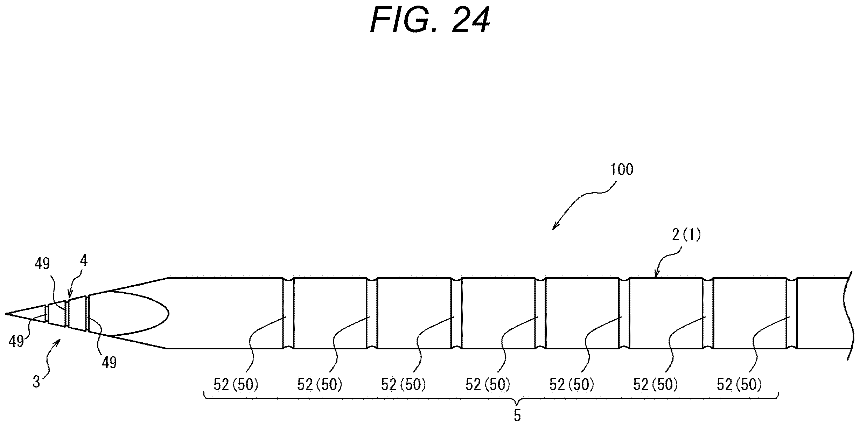

Fig. 24 illustrates a side view of a puncture needle 100 according to the present embodiment. As illustrated in Fig. 24, in the puncture needle 100 of the present embodiment, the needle tip portion 3 has a conical shape. A first structure 4 is formed at a distal end portion of the needle tip portion 3. The first structure 4 may be formed by, for example, arranging annular grooves 49 adjacent to each other in an axial direction.

In the example illustrated in Fig. 24, a case where a plurality of reflection structure portions 50 formed of one annular groove 52 is arranged at equal intervals along the axial direction in a second structure 5 is illustrated.

As described above, it is possible to provide a puncture needle capable of, while securing echo visibility of the puncture needle, further grasping a puncture length.

Note that the configurations disclosed in the above-described embodiments (including the other embodiments, the same applies hereinafter) can be applied in combination with configurations disclosed in other embodiments as long as there is no contradiction, and the embodiments disclosed in the present specification are examples, and the embodiments of the present disclosure are not limited thereto, and can be appropriately modified within a scope not departing from the object of the present disclosure.

### Industrial Applicability

The present disclosure is applicable to a puncture needle.

### Reference Signs List

- 1: Main body portion
- 100: Puncture needle
- 2: Body portion
- 20: Outer surface (Outer peripheral surface)
- 29: Inner surface
- 29a: Exposed inner surface
- 3: Needle tip portion
- 30: Outer surface
- 31: Planar portion
- 3A: Blade
- 4: First structure (First ultrasound reflection structure)
- 41: Transverse groove
- 42: Intersection groove
- 49: Annular groove
- 5: Second structure (Second ultrasound reflection structure)
- 50: Reflection structure portion
- 50a: First reflection structure portion
- 50b: Second reflection structure portion
- 51: Spiral groove
- 52: Annular groove
- 53: Dotted-line circular groove
- 54: Annular oblique line groove
- 54a: Oblique line groove
- 55: Arc array groove
- 55a: Groove
- 6: Blade surface portion
- 60: Blade surface
- 61: First blade surface portion
- 62: Second blade surface portion
- 63: Third blade surface portion
- 69: Opening
- 6A: Blade
- 71: V-shaped groove
- 72: Circular recess
- 73: Groove
- 74: Triangular recess
- 75: Groove
- 76: Groove
- 77: Groove
- G: Axial center
- L1: Extension length
- L2: Separation distance
- L3: Separation distance
- P: Boundary point
- Q: Center
- T: Vertex
- W: Ultrasound
- W1: Reflected wave
- W2: Reflected wave
- W3: Reflected wave
- W4: Reflected wave
- W5: Reflected wave
- W6: Reflected wave

## Claims

1. A puncture needle comprising:
a rod-shaped main body portion;
a first ultrasound reflection structure formed in the main body portion; and
a second ultrasound reflection structure formed in the main body portion and arranged on a proximal end side in an axial direction of the main body portion with respect to the first ultrasound reflection structure,
the second ultrasound reflection structure being arranged separated from the first ultrasound reflection structure in the axial direction.

2. The puncture needle according to claim 1, wherein
the main body portion includes a rod-shaped body portion and a needle tip portion arranged at a distal end of the body portion,
the first ultrasound reflection structure is arranged at the needle tip portion, and
the second ultrasound reflection structure is arranged at the body portion.

3. The puncture needle according to claim 2, wherein the first ultrasound reflection structure is arranged at a distal end portion of the needle tip portion.

4. The puncture needle according to claim 2 or 3, wherein the second ultrasound reflection structure is a spiral groove formed on an outer peripheral surface of the body portion.

5. The puncture needle according to any one of claims 2 to 4, wherein the needle tip portion has a pyramid shape.

6. The puncture needle according to any one of claims 2 to 5, wherein
the body portion is formed in a cylindrical shape, and
the needle tip portion has an opening that communicates an internal space and an external space of the body portion.
